# EUROPÄISCHE PATENTSCHRIFT

(11) **EP 4 100 118 B1**
(45) Veröffentlichungstag und Bekanntmachung des Hinweises auf die Patenterteilung: **20.08.2025**
(21) Anmeldenummer: 21703912.2
(22) Anmeldetag: 05.02.2021
(51) Int. Cl.: A61N 5/10, A61B 90/00

(54) **IMPLANTIERBARE MARKIERUNGSKÖRPER FÜR DIE BRUSTTHERAPIE**
IMPLANTABLE MARKER BODY FOR BREAST TREATMENT
CORPS DE MARQUAGE IMPLANTABLE POUR RÉALISER UN TRAITEMENT MAMMAIRE

(30) Priorität: 06.02.2020 DE 102020000762
(43) Veröffentlichungstag der Anmeldung: 14.12.2022
(73) Patentinhaber: SOMATEX Medical Technologies GmbH, 14199 Berlin (DE)
(72) Erfinder: KRAUSE, Thorsten, 14199 Berlin (DE); DREWES, Nils, 14199 Berlin (DE); MELZER, Sophie, 14199 Berlin (DE)
(74) Vertreter: Hoyng Rokh Monegier B.V.
(86) Internationale Anmeldenummer: PCT/EP2021/052864
(87) Internationale Veröffentlichungsnummer: WO 2021/156474

(56) Entgegenhaltungen:
- WO-A1-01/08578
- US-A1- 2009 000 629
- US-A1- 2013 289 389
- US-A1- 2017 333 233

## Beschreibung

Die Erfindung betrifft implantierbare Markierungskörper - kurz: Marker - für die Brusttherapie, insbesondere für eine Brust-Strahlentherapie.

Ein Bruststrahlentherapiemarker dient der intraoperativen Markierung des Brusttumorbetts für eine postoperative Strahlentherapie.

An einen Bruststrahlentherapiemarker wird die Anforderung gestellt, dass der Marker resistent gegen Strahlung sein muss und keine Veränderungen durch die Strahlentherapie erfahren darf. Der Marker soll das Tumorbett zuverlässig für die anschließende Bestrahlungsplanung und -behandlung durch integrierte röntgendichte bzw. unter Röntgenstrahlung basierter Bildgebung sichtbare Markierungselemente möglichst exakt lokalisieren, um das Volumen bestrahlten Körpergewebes so gering wie möglich zu halten und das umliegende gesunde Gewebe zu schonen. Dies bedeutet auch, dass der Marker nicht dislozieren darf.

Anhand der Lage der Markierungselemente des implantierten Markers kann die Dosisplanung der Strahlentherapie ausgerichtet werden. Je weiter die Markierungselemente voneinander entfernt sind desto genauer kann die Dosisplanung erfolgen.

Darüber hinaus sollte sich der Marker "weich" anfühlen, damit der Marker postoperativ nicht in der Brust tastbar ist. Zudem sollte der Marker auch unter MR Bildgebung verwendbar sein. Die MR Bildartefakte sollten maximal 3 mal, besser 1,5 mal oder im Optimalfall genauso groß sein wie der Marker selbst. Die MR Artefaktgröße wird durch die Materialien bestimmt. Insbesondere durch metallische Werkstoffe aus denen vorzugsweise die Markierungselemente bestehen.

US 2013/0289389 A1 beschreibt eine implantierbare Gewebe markie Vorrichtungen zur Platzierung in einem weichen Gewebe durch einen chirurgischen Einschnitt Punkt die Vorrichtung kann einen Bio - absorbierbare Körper in der Form einer Spirale umfassen und eine Kugelform für die Vorrichtung definieren, wobei die Spirale eine Längsachse aufweist und Bindungen der Spirale voneinander in einer Rechnung beabstandet sind entlang der Längsachse.

### ZUSAMMENFASSUNG

Während die Erfindung in dem unabhängigen Anspruch definiert ist, ergeben sich weitere Aspekte der Erfindung aus den abhängigen Ansprüchen, den beigefügten Figuren und der nachfolgenden Beschreibung.

Der Erfindung liegt die Aufgabe zugrunde, einen oder verschiedene Markierungskörper zu schaffen, die die vorgenannten Anforderungen möglichst gut erfüllen.

Erfindungsgemäß wird diese Aufgabe durch einen Markierungskörper (Marker) mit den Merkmalen des Anspruchs 1 gelöst. Dementsprechend wird der Markierungskörper von einem Schlauch oder einem wenigstens abschnittsweise schlauchartigen Körper aus einem weich-elastischem Material gebildet, der mehrere radioopake Markierungselemente trägt. Mit "weich-elastisch" wird ein Schlauch bezeichnet, der einer äußeren, verformenden Kraft kaum Widerstand entgegensetzt, in Abwesenheit von äußeren Kräften jedoch zu seiner ursprünglichen Form zurückkehrt.

Der wenigstens abschnittsweise schlauchartige Körper weist zwei freie Enden auf, die lösbar miteinander verbunden werden können. Vorzugseise ist hierzu eine Steckverbindung vorgesehen. Wenn die freien Längsenden des wenigstens abschnittsweise schlauchartigen Körpers miteinander verbunden sind, entsteht ein Ring (nachfolgend auch als "Markerring" oder "Schlauchring-Marker" bezeichnet), der den Markierungskörper bildet. Die elastischen Kräfte, die der Markierungskörper einer äußeren Kraft entgegensetzt, sind vorzugsweise kleiner als 1N auf 1mm Komprimierungsweg bei einem Markerringaußendurchmesser von 3 cm.

Der wenigstens abschnittsweise schlauchartige Körper weist vorzugsweise wenigstens an einem seiner freien Enden ein Lumen auf, in das ein anderes freies Ende des wenigstens abschnittsweise schlauchartigen Körpers oder ein Verbindungselement eingesteckt werden kann, um die beiden freien Enden des wenigstens abschnittsweise schlauchartigen Körpers miteinander zu verbinden. Die Verbindung ist vorzugsweise eine Klemmverbindung, bei der wenigstens eines der miteinander verbundenen freien Enden des wenigstens abschnittsweise schlauchartigen Körpers in Vergleich zu dessen unverbundenem Zustand radial zumindest geringfügig aufgeweitet ist, um durch elastische Rückstellkräfte die Klemmwirkung zu erzielen. Die Verbindung ist vorzugsweise so konfiguriert, dass die Kraft, die nötig ist, um die Verbindung wieder zu lösen, mindestens 2 N beträgt, besonders bevorzugt auch mindestens 20 N

Vorzugsweise weist der wenigstens abschnittsweise schlauchartige Körper ein durchgehendes Lumen auf oder zwei oder mehr Lumina, die sich zusammengenommen über mehr als die Hälfte der Gesamtlänge des wenigstens abschnittsweise schlauchartigen Körpers erstrecken. Dies erlaubt es, den wenigstens abschnittsweise schlauchartigen Körper dort, wo er ein Lumen aufweist, zu kürzen und im Ergebnis einen kürzeren wenigstens abschnittsweise schlauchartigen Körper zu schaffen, dessen freie Enden aufgrund des Lumens zusammengesteckt werden können, um einen Markierungskörper mit an einen jeweiligen Implantationsort angepassten Abmessungen zu schaffen.

Wenn der wenigstens abschnittsweise schlauchartige Körper in seinem Ausgangszustand mit unverbundenen freien Enden im entspannten Zustand grade ist, kann der wenigstens abschnittsweise schlauchartige Körper durch Verbinden seiner beiden freien Enden miteinander zu einem elastischen Ring geformt werden und es entsteht ein Schlauchring-Marker. Dieser hat wenigstens annähernd eine Kreisform, wenn die Querschnitte des wenigstens abschnittsweise schlauchartigen Körpers gleiche oder ähnliche Flächenträgheitsmomente aufweisen und der wenigstens abschnittsweise schlauchartige Körper - mit Ausnahme der Markierungselemente - über seine Länge von dem gleichen Material oder gleichen Materialien gebildet ist.

Ein derartiger Schlauchring-Marker ermöglicht eine Mehrpunktmarkierung für den CT und gleichzeitig lässt er sich durch Zurechtschneiden patientenindividuell auf jede Größe einstellen (zusammenstecken).

Existierende Produkte für diese Indikation erfordern, dass das Krankenhaus verschiedene Größen des Markers vorrätig hat, je nach Größe des Tumorbetts. Für den Schlauchring Marker muss nur eine Größe vorrätig gehalten werden, die dann intraoperativ auf die gewünschte Länge angepasst werden kann.

Der wenigstens abschnittsweise schlauchartige Körper besteht vorzugsweise aus einem weichen bioresorbierbaren Polymer (z.B. PLA, PLLA, Polyglycolsäure, Polycaprolacton, Poly-p-dioxanon, ε-Caprolacton, Evonik Resomer, oder ähnlich). Das Polymer ist vorzugsweise so konfiguriert, dass es für mindestens 6 Monate im Gewebe stabil ist und anschließend resorbiert. Alle 2 bis 3 cm befinden sich innerhalb des Schlauchs (oder außerhalb festgeklemmt) radioopake, vorzugsweise metallische Markierungselemente, die dann im CT-Bild sichtbar sind. Bevorzugte biokompatible Metalle für die Markierungselemente sind Gold, Titan, Tantal oder Nitinol. In ein Lumen des Markierungskörpers können dazu beispielsweise kleine Metallzylinder eingesetzt werden (z.B. ca. Ø1,5 mm x 3 mm). Auch andere geometrische Formen sind denkbar, solange sie in oder an dem wenigstens abschnittsweise schlauchartigen Körper nicht dislozieren.

Die Gesamtlänge des v vorzugsweise zwischen 6 cm, 7,5 cm, 15 cm 20 cm und 30 cm. Bei einer Länge von 30 cm ergibt sich nach Zusammenstecken der Längsenden ein maximaler Durchmesser von ca. 9,5 cm, was auch für sehr große Tumorbetten nach Lumpektomie ausreichend ist. Der Außendurchmesser des wenigstens abschnittsweise schlauchartigen Körpers beträgt vorzugsweise zwischen 1mm und 5mm.

Die Verbindung der freien Enden des wenigstens abschnittsweise schlauchartigen Körpers kann entweder nach Art einer Steckbuchse erfolgen (Variante 1), bei der dasjenige freie Längsende, an dem der wenigstens abschnittsweise schlauchartige Körper abgeschnitten wurde, in dessen anderes, im Durchmesser etwas erweiterte Längsende gesteckt wird. In einer alternativen Variante 2 haben beide Längsenden des wenigstens abschnittsweise schlauchartigen Körpers den gleichen Durchmesser und werden mittels eines Verbindungselements, vorzugsweise eines Verbindungsstifts, zusammengesteckt. Dieser Verbindungsstift besteht vorzugsweise aus einem ebenfalls bioresorbierbaren Kunststoff, der härter ist, als das Material, auch dem der wenigstens abschnittsweise schlauchartige Körper gebildet ist. Alternativ kann der Verbindungsstift metallisch sein und somit ebenfalls als im CT-Bild sichtbares Markierungselement dienen. Der wenigstens abschnittsweise schlauchartige Körper besitzt vorzugsweise an seinen beiden Längsenden ein Lumen, in das der Verbindungsstift gesteckt werden kann, um die beiden Längsenden des wenigstens abschnittsweise schlauchartigen Körpers miteinander zu verbinden.

Um eine Migration des Markierungskörpers zu verhindern, wird der Markierungskörperdurch Ösen (optional) oder Schlaufen im Tumorbett eingenäht. Der wenigstens abschnittsweise schlauchartige Körper ist flexibel und passt sich der Form des Tumorbettes an. Dadurch sollte der Markierungskörper postoperativ nicht in der Brust tastbar sein. Die flache Form ermöglicht Anpassungen an übliche onkoplastische Operationstechniken.

Im Auslieferungszustand soll in einer Ausführungsform der wenigstens abschnittsweise schlauchartige Körper schon in Ringform bzw. einer Form vorliegen, die dem Implantationszustand nahekommt. Der Durchmesser des ringförmig vorgeformten wenigstens abschnittsweise schlauchartigen Körpers ist vorzugsweis etwas kleiner zu wählen als der kleinste Markierungskörper, der aus dem wenigstens abschnittsweise schlauchartigen Körper gebildet werden soll, so dass sich die Endabschnitte des wenigstens abschnittsweise schlauchartigen Körpers überlappen, bevor der wenigstens abschnittsweise schlauchartige Körper passend gekürzt wird und dessen Längsenden miteinander verbunden werden.

In weiteren Varianten des Markierungskörpers ist dieser dazu ausgebildet, dass mehrere wenigstens abschnittsweise schlauchartige Körper zu einem Markierungskörper verbunden werden können oder verbunden sind.

Hierzu können Verbindungselemente mit mehreren freien Enden vorgesehen sein, die in Lumina an den freien Längsenden der wenigstens abschnittsweise schlauchartigen Körper gesteckt werden können, um auf diese Weise freie Längsenden mehrerer wenigstens abschnittsweise schlauchartiger Körper zu einem Markierungskörper miteinander verbinden zu können.

Gemäß einer weiteren Variante wird der Markierungskörper von zwei Schlauch-Ring Markern gebildet. Beispielsweise können zwei Schlauchring-Marker ineinandergesteckt werden (z.B. um 90° gedreht), um eine Art Ball zu kreieren. Die nun dreidimensionale Form des Markers würde die Tumorhöhle ausfüllen bzw. offenhalten und verhindern, dass die Brust an dieser Stelle einfällt. Das Gewebe kann in den Marker einwachsen. Neben einer akkuraten Bestrahlungsplanung würde der Marker den kosmetischen Aspekt der Rekonstruktion unterstützen.

Ein weiterer Aspekt ist ein modulares System, bei dem ein zentrales Element - der im wesentlichen schlauchartige Körper - mit sich selbst verbunden werden kann, bis die gewünschte Länge erreicht wird. Die einzelnen im wesentlichen schlauchartigen Körper können hierfür entsprechend kürzer ausgebildet sein. So kann der Arzt mehrere Elemente zusammenstecken, ohne dass er etwas zerschneiden muss. Ein Zerschneiden des Produktes entfällt, und ggf. wird dadurch Ausschuss erspart der durch das Zerschneiden produziert wird.

Gemäß einer weiteren Variante besitzt der im wesentlichen schlauchartige Körper an seinen beiden Längsenden Schlaufen, durch die der im wesentlichen schlauchartige Körper selbst hindurchgeführt ist, so dass sich ein in seiner Weite verstellbarer Ring ergibt, der somit an die Größe des Tumorbetts anpassbar ist. Der stufenlos einstellbare Durchmesser dieses Markierungskörpers kann vorzugsweise durch einen nicht dargestellten Klemmmechanismus an mindestens einer Schlaufe (32 oder 34) oder in Stufen bei denen ein Element an der Schlaufe mittels Formschluss an den Bereich zwischen den Schlaufen fixiert werden.

Nachfolgend sind geeignete Materialien für den wenigstens abschnittsweise schlauchartigen Körper und die Verbindungselemente aufgeführt: Chitosan, Chitin und deren Derivate, PGA (Polyglycolide /polyglykolsäure), Dextran, PLA (Polylactid / Polymilchsäure), PLLA (Poly-L-Lactid), PDLA (Poly-D/L-Lactid), PLDLLA (Poly-L-co-D/L-Lactid), PLGA (Poly lactid-co-glycolid), PCL (Poly-ε-Caprolacton), PEG (Poly ethylene glycol), PVA (Poly vinyl alcohol), PDO (Poly-p-dioxanon), PHA (Polyhydroxyalkanoate) und PPG (Poly propylen glycol)

Des Weiteren sind auch folgende nichtresorbierende Materialien geeignet: Silikon, PA (Polyamid), PPG (Poly propylen glycol), Pebax, Polyurethan, PE (Polyethylen), LDPE und PVDF (Polyvinylidenflourid)

Geeignete Materialien für die Markierungselemente sind: Gold, Platin, Nitinol, Tantal, Titan, Kunststoff mit Bariumsulfat.

Ein geeignetes Material für resorbierbare Markierungselemente ist Magnesium.

Die Erfindung soll nun anhand von Ausführungsbeispielen mit Bezug auf die Figuren näher erläutert werden. Von den Figuren zeigt:
- Fig. 1a - 1e:: zeigen einen schlauchartigen Körper (Fig. 1a) und einen daraus zusammengesetzten erfindungsgemäßen Markierungskörper (Fig. 1e) sowie Möglichkeiten, wie die freien Längsenden des schlauchartigen Körpers miteinander verbunden werden können (Fig. 1b und 1c) und wie der schlauchartige Körper interoperativ gekürzt werden kann (Fig. 1d);
- Fig. 2a - 2c:: zeigt eine alternative Variante für einen schlauchartigen Körper, der zu einem erfindungsgemäßen Markierungskörper zusammengesteckt werden kann, wobei der Markierungskörper aus einer Mehrzahl an schlauchartigen Körpern besteht, die in Längsrichtung zusammengesteckt sind;
- Fig. 3a -3c:: zeigt in Fig. 3a einen schlauchartigen Körper und radiopaque Markierungselemente für den schlauchartigen Körper sowie in Fig. 3b und 3c Detaildarstellungen für zwei verschiedene Varianten zum Verbinden der freien Längsenden des schlauchartigen Körpers;
- Fig. 4a - 4c:: verschiedene erfindungsgemäße Markierungskörper, die durch unterschiedlich lange schlauchartige Körper gebildet sind;
- Fig. 5a - 5c:: in Fig. 5a, einen schlauchartigen Körper sowie in Figuren 5 b und c verschiedene Varianten, um die Längsenden des schlauchartigen Körpers miteinander zu verbinden, um einen erfindungsgemäßen Markierungskörper zu bilden;
- Fig. 6a - 6c:: verschiedene Verbindungselemente zum Verbinden freier Enden eines oder mehrerer schlauchartiger Körper zu einem erfindungsgemäßen Markierungskörper;
- Fig. 7a und 7b:: zeigen zwei verschiedene Varianten eines erfindungsgemäßen Markierungskörpers, der mit den in Fig. 6 abgebildeten Verbindungselementen in Verbindung mit drei bzw. zwei schlauchartigen Körpern gebildet werden kann;
- Fig. 8:: eine Variante eines schlauchartigen Körpers für einen erfindungsgemäßen Markierungskörper mit längsgestreckten Markierungselementen und Sollbruchstellen;
- Fig. 9:: eine Variante eines erfindungsgemäßen Markierungskörpers, der durch Ösen an seinen beiden Längsenden in der Weite einstellbar und so an einen Implantationsort anpassbar ist;
- Fig. 10a und 10b:: zwei Varianten eines Markierungskörpers gemäß Fig. 9 mit unterschiedlicher Ausrichtung und Ausführung der als Hülsen ausgebildeten Ösen an den Längsenden des schlauchartigen Körpers;
- Fig. 11a und 11b:: zeigen in Fig. 11a zwei schlauchartige Körper und ein Verbindungselement, die zu den Markierungskörpern gemäß Fig. 10 zusammengesetzt werden können, und Fig. 11b illustriert, wie zwei schlauchartige Körper
- Fig. 12:: gemäß Fig. 11a zunächst zusammengeschoben und anschließend an ihren Längsenden mit dem Verbindungselement zu einem geschlossenen Markierungskörper zusammengesteckt werden können; eine Variante eines Körpers für einen Markierungskörper mit Verbindungselementen, die nach Art eines Puzzles miteinander verbunden werden können;
- Fig. 13:: eine erste Variante eines Markierungskörpers mit einem als gebogene Nadel ausgeführten Längsendes;
- Fig. 14:: eine zweite Variante eines Markierungskörpers mit einem als gebogene Nadel ausgebildeten Längsendes;

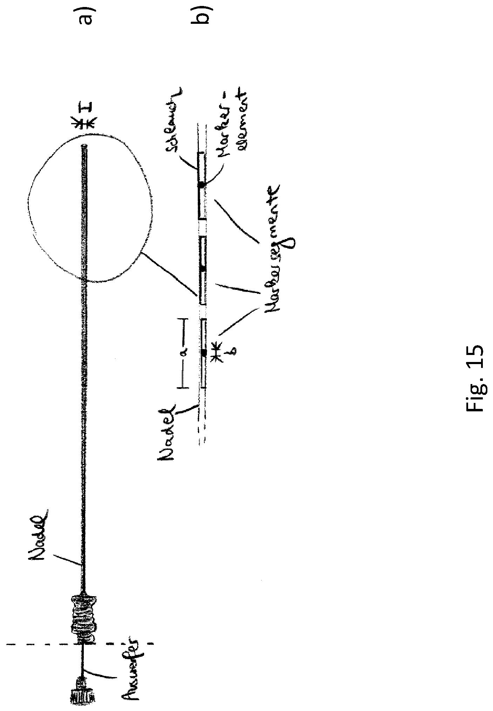

- Fig. 15a und 15b:: ein alternatives Produktkonzept mit einer Vielzahl von Markierungselementen, die in einer Nadel vorgehalten werden;

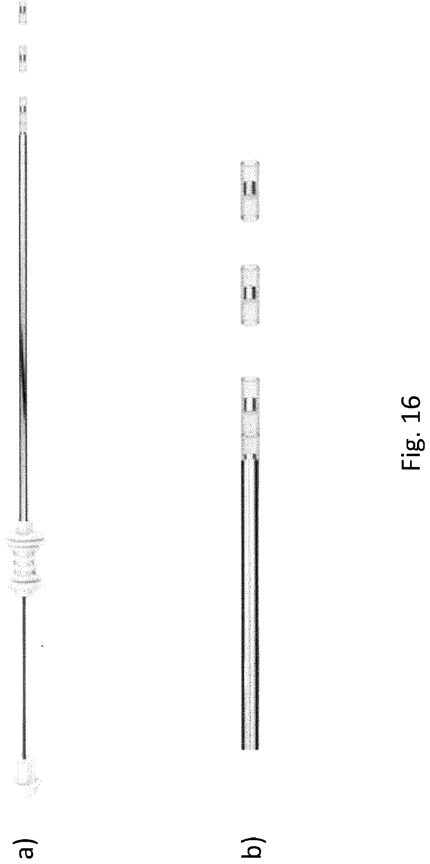

- Fig. 16a und 16b:: weitere Darstellungen des Produktkonzepts aus Fig. 15;
- Fig. 17a und 17b:: ein alternatives Produktkonzept in Form eines flexiblen Netzes;
- Fig. 18a und 18b:: ein alternatives Produktkonzept in Formeines komprimierbaren Balls;
- Fig. 19:: ein alternatives Produktkonzept in Form einer 3D-Matrix

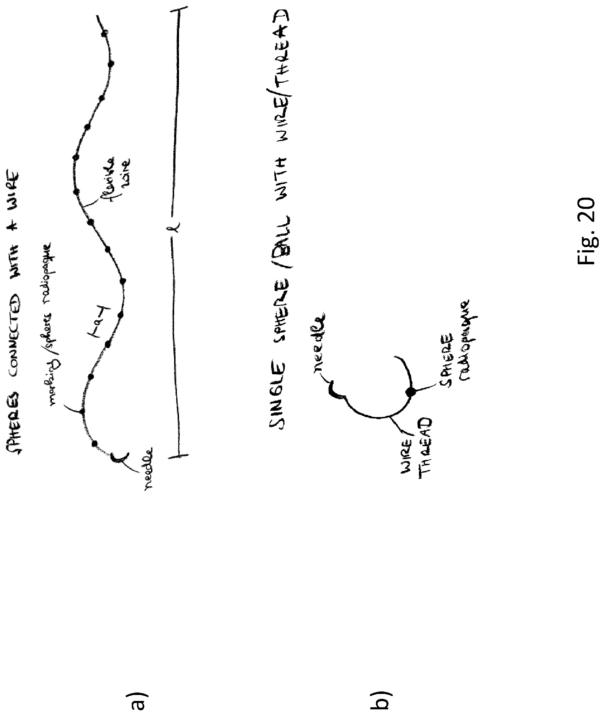

- Fig. 20a und 20b:: ein alternatives Produktkonzept in Form resorbierbarer Magnesiumkugeln, verbunden mit einem resorbierbaren Faden oder Magnesiumdraht;
- Fig. 21:: ein alternatives Produktkonzept in Form eines Strahlentherapiefadens mit Metallsegmenten; und
- Fig. 22a - 22d:: alternative Produktkonzepte in Form eines Silikonmarkers oder eines Hydrogelmarkers.

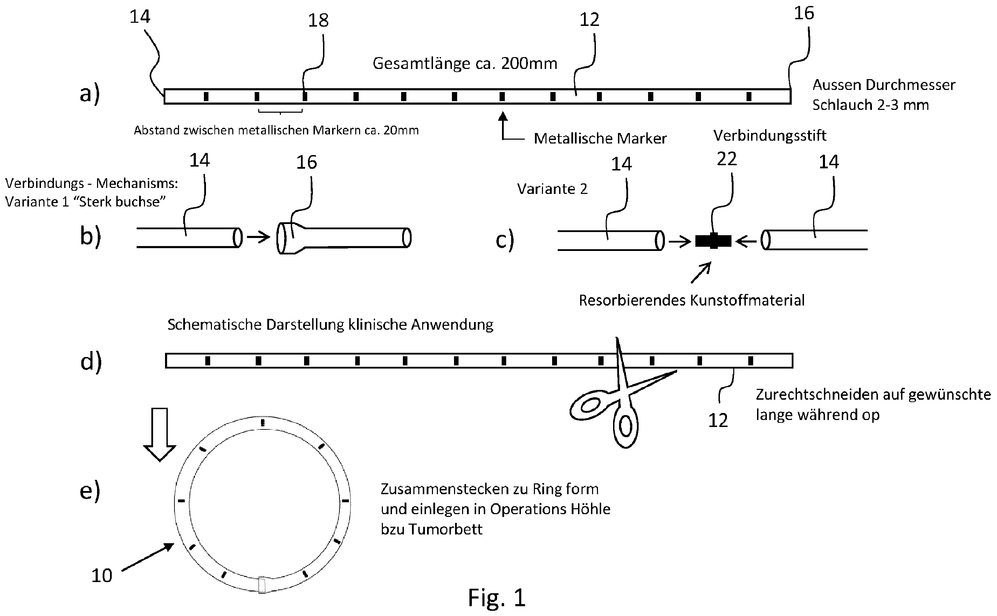

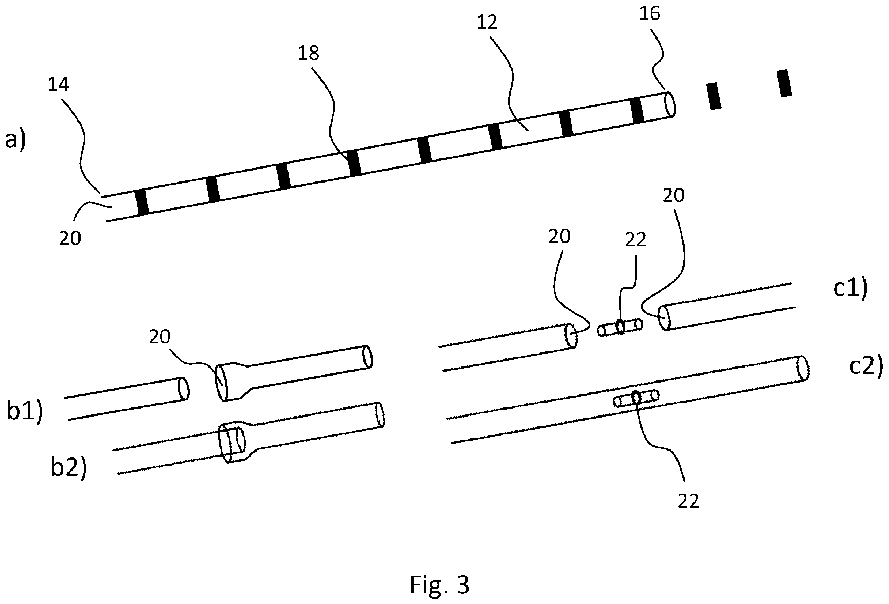

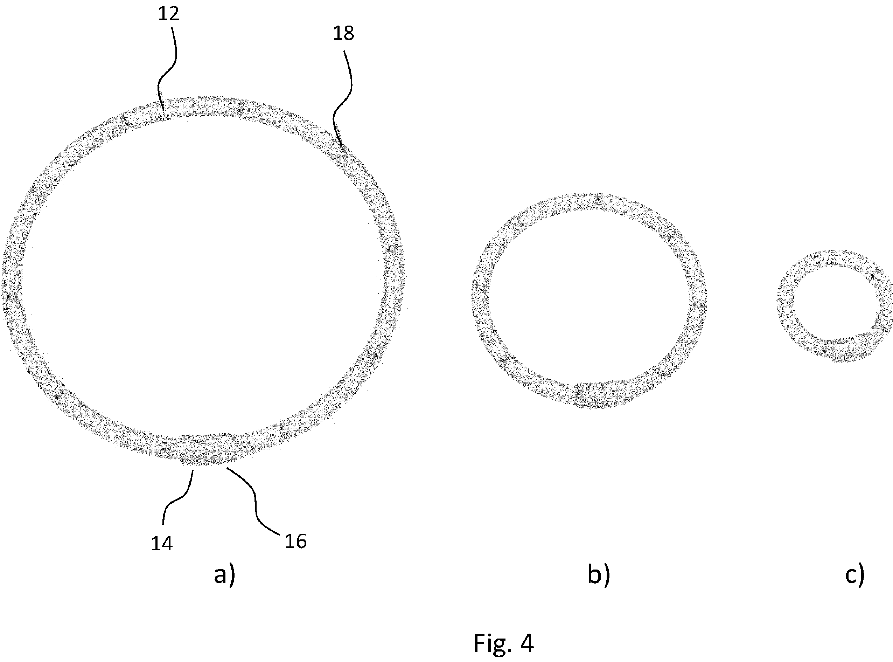

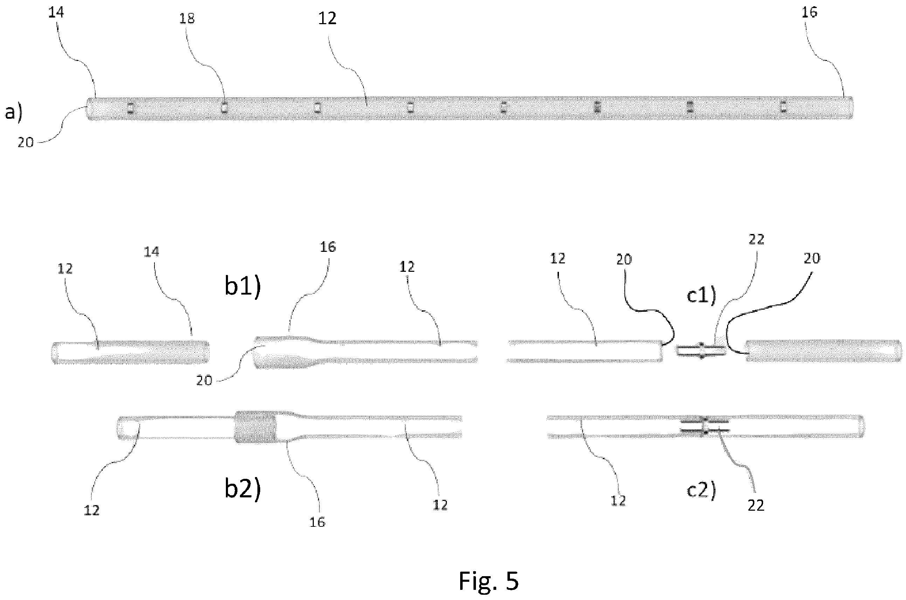

Ein erfindungsgemäßer Markierungskörper 10 (siehe Figuren 1e und Figuren 4a-c) ist gemäß einer ersten Variante von einem schlauchartigen Körper 12 gebildet, dessen freie Längsenden 14 und 16 miteinander verbunden sind, sodass der Markierungskörper 10 zumindest annähernd die Form eines Kreisrings hat. Der schlauchartige Körper 12 trägt eine Vielzahl von Markierungselementen 18, die auf den schlauchartigen Körper 12 aufgebracht oder in den schlauchartigen Körper 12 eingebracht sein können; siehe **Figuren 1-5****.**

Der schlauchartige Körper 12 kann als Schlauch mit einem durchgehenden Lumen ausgebildet sein oder er kann abschnittsweise Lumina aufweisen - also kein durchgehendes Lumen.

Wie insbesondere Fig. 1d zeigt, kann der schlauchartige Körper 12 gekürzt werden, um auf diese Weise Markierungskörper 10 mit unterschiedlichem Durchmesser erzeugen zu können, wie dies in den Figuren 4a-c dargestellt ist.

Vorzugsweise wird der schlauchartige Körper 12 dort gekürzt, wo er ein Lumen aufweist. Das jeweilige Lumen 20 des schlauchartigen Körpers 12 dient dazu, die Längsenden 14, 16 des schlauchartigen Körpers 12 miteinander verbinden zu können. Gemäß einer ersten Variante (siehe Figuren 1b, 3b, 4a-c und 5b) ist eines der Längsenden 14 oder 16 des schlauchartigen Körpers 12 aufgeweitet, sodass das jeweils andere Längsende 16 bzw. 14 in das entsprechend aufgeweitete Längsende eingesteckt werden kann, um die beiden Längsenden 14, 16 des schlauchartigen Körpers 12 auf diese Weise miteinander zu verbinden und den erfindungsgemäßen Markierungskörper 10 zu erzeugen.

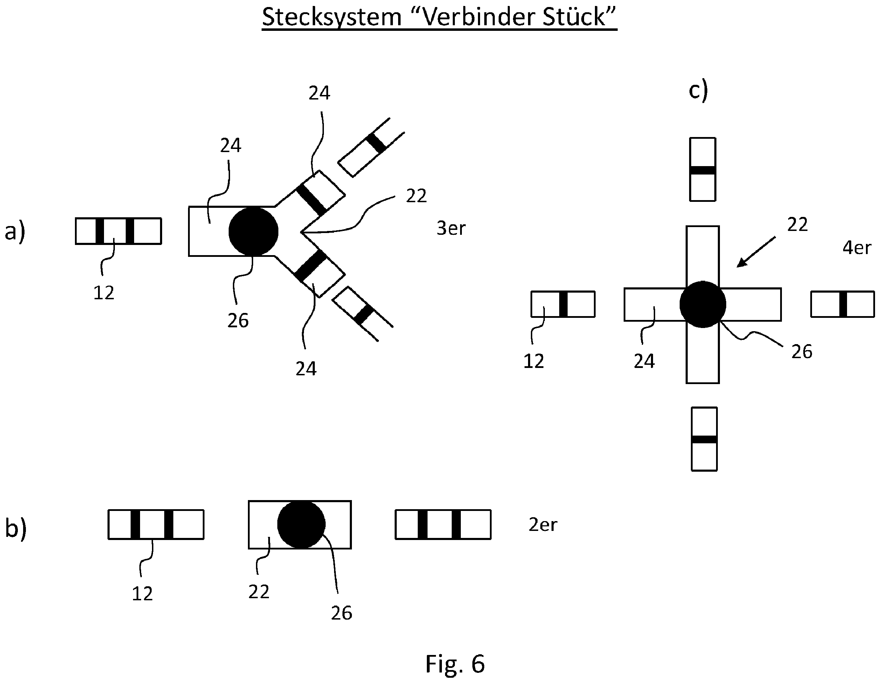

Alternativ können die Lumina 20 an den beiden Längsenden 14 und 16 des schlauchartigen Körpers 12 auch den gleichen Innendurchmesser haben. In diesem Falle kann ein gesondertes Verbindungelement 22 - beispielsweise ein Verbindungsstift - vorgesehen sein, der in die Lumina 20 an den beiden Längsenden 14 und 16 des schlauchartigen Körpers 12 eingesteckt werden kann, um diese Längsenden 14 und 16 des schlauchartigen Körpers miteinander verbinden zu können; siehe beispielsweise Fig. 1c, 3, und 5c. Die Verbindungelemente 22 können als Verbindungsstifte ausgebildet sein, so wie dies in den Figuren 1c, 3c und 5c dargestellt ist. Alternativ können die Verbindungelemente 22 aber selbst mehr als zwei Verbindungsenden 24 aufweisen, beispielsweise drei Verbindungsenden 24 (siehe **Fig. 6a****)** oder vier Verbindungsenden 24 (siehe **Fig. 6c****).** Die Verbindungsenden 24 können jeweils entweder als Hülsen ausgebildet sein, in die die freien Längsenden 14 oder 16 eines schlauchartigen Körpers 12 eingesteckt werden können, oder die freien Verbindungsenden 24 können als Stifte ausgebildet sein, die in die jeweiligen Lumina 20 an den freien Längsenden 14 oder 16 des schlauchartigen Körpers 12 gesteckt werden können.

Auch können die Verbindungelemente 22 jeweils mindestens ein Markerelement 26 aufweisen. Alternativ können die Verbindungelemente selbst auch radiopaque ausgebildet sein, sodass kein gesondertes Markerelement 26 erforderlich ist.

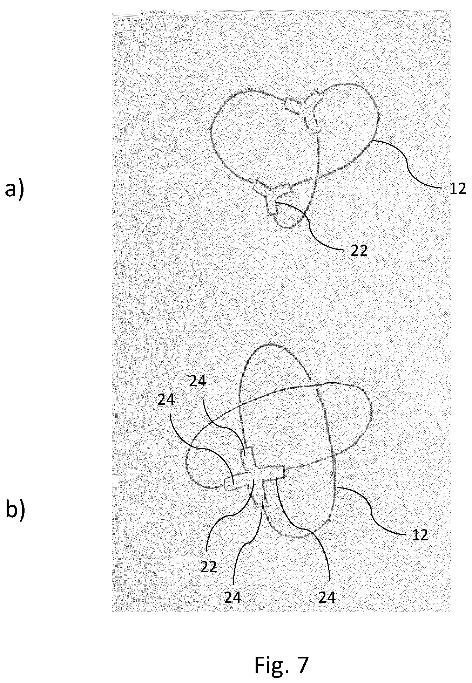

Mit den in Fig. 6a oder in Fig. 6c dargestellten Verbindungelementen 22 mit mehr als zwei Verbindungsenden 24 können solche Markierungskörper erzeugt werden, wie sie skizzenhaft in den **Figuren 7a** und **7b** dargestellt sind. Beispielsweise kann aus zwei Verbindungselementen 22 mit jeweils drei Verbindungsenden 24 und drei schlauchartigen Körpern 12 ein Markierungskörper zusammengesteckt werden, wie er in Fig. 7a abgebildet ist. Um die Größe eines derartigen Markierungskörpers 10' an den jeweiligen Implantationsort anpassen zu können, können auch hier schlauchartigen Körper 12 entsprechend gekürzt werden.

Mit einem Verbindungselement 22 mit vier Verbindungsenden 24 kann in Verbindung mit zwei schlauchartigen Körpern 12 ein Markierungskörper 10" zusammengesteckt werden, der beispielsweise die in Fig. 7b skizzenhaft dargestellte Form hat. Auch hier können die schlauchartigen Körper 12 jeweils entsprechend gekürzt werden, um den Markierungskörper 10" an den jeweiligen Implantationsort anzupassen. Nicht dargestellt ist eine Abwandlung des in Fig. 7b dargestellten Markierungskörpers 10", bei dem ähnlich wie bei dem in Fig. 7a dargestellten Beispiel zwei Verbindungselemente 22 mit jeweils vier Verbindungsenden 24 vorgesehen sind.

Auch, wenn beispielsweise in den Fig. 7a und 7b keine Markierungselemente 18 gezeigt sind, weisen die schlauchartigen Körper 12 solche Markierungselemente auf. Tatsächlich können die schlauchartigen Körper 12 bei den Markierungskörpern 10' und 10" gemäß Figuren 7a und 7b genauso aussehen, wie die in Figuren 1a, 3a und 5 a dargestellten schlauchartigen Körper 12.

Die schlauchartigen Körper 12 sind vorzugsweise aus einem bioresorbierbarem Kunststoff hergestellt. Infrage kommen die nachfolgend aufgelisteten bioresorbierbaren Werkstoffe: Chitosan, Chitin und deren Derivate, PGA (Polyglycolide /polyglykolsäure), Dextran, PLA (Polylactid / Polymilchsäure), PLLA (Poly-L-Lactid), PDLA (Poly-D/L-Lactid), PLDLLA (Poly-L-co-D/L-Lactid), PLGA (Poly lactid-co-glycolid), PCL (Poly-ε-Caprolacton), PEG (Poly ethylene glycol), PVA (Poly vinyl alcohol), PDO (Poly-p-dioxanon), PHA (Polyhydroxyalkanoate) und PPG (Poly propylen glycol).

Des Weiteren sind auch folgende nichtresorbierende Materialien geeignet: Silikon, PA (Polyamid), PPG (Poly propylen glycol), Pebax, Polyurethan, PE (Polyethylen), LDPE und PVDF (Polyvinylidenflourid)

Die Markierungselemente 18 bestehen vorzugsweise aus einem radiopaquen Metall, wie Gold, Platin, Nitinol, Tantal, Titan, Kunststoff mit Bariumsulfat.

Ein geeignetes Material für resorbierbare Markierungselemente 18 ist Magnesium.

Die Markierungselemente 18 können außen auf dem wenigstens abschnittsweise schlauchförmigen Körper aufgeklemmt sein. Alternativ können die Markierungselemente 18 in entsprechende Lumina 20 des schlauchartigen Körpers 12 eingesetzt sein oder in das Trägermaterial des schlauchartigen Körpers 12 eingegossen sein, indem das Trägermaterial beispielsweise im Spritzgießverfahren um die Markierungselemente herumgespritzt wird.

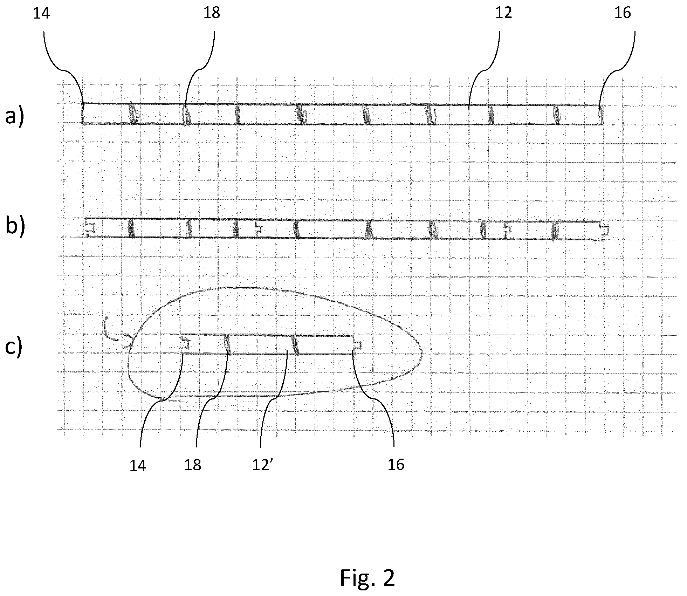

Ein Markierungskörper 10 kann auch aus mehreren schlauchartigen Körpern 12 zusammengesetzt sein, die an ihren Längsenden miteinander verbunden sind. Gemäß einer Variante ist vorgesehen, dass die schlauchartigen Körper relativ kurz sind, sodass sie zum Anpassen an einen Implantationsort nicht gekürzt zu werden brauchen, sondern vielmehr mehrere schlauchartige Körper 12 zu einem Markierungskörper 10 zusammengesteckt werden können. Dies ist beispielhaft in Figuren 2b und 2c dargestellt. Fig. 2b zeigt dabei einen schlauchartigen Körper 12, der aus mehreren schlauchartigen Körpern 12 zusammengesetzt ist, wie sie beispielsweise in Fig. 2c gezeigt sind.

Wie aus Fig. 2 ebenfalls hervorgeht, können die schlauchartigen Körper 12 auch an einem Längsende 14 nur ein kurzes Lumen 20 aufweisen und am jeweils anderen Längsende 16 einen entsprechenden stiftartigen Vorsprung 28, der in das Lumen 20 eingesteckt werden kann, um eine Verbindung zwischen Längsenden 14 und 16 des schlauchartigen Körpers 12 herzustellen. In diesem Falle ist kein gesondertes Verbindungselement erforderlich und es muss auch kein aufgeweitetes Längsende vorgesehen sein. Es versteht sich, dass solche kurzen schlauchartigen Körper 12, wie sie in Fig. 2c dargestellt sind, auch Längsenden 14 und 16 aufweisen können, wie sie beispielsweise in den Figuren 1b, 1c, 3b, 3c, 5b und 5c dargestellt sind. Dementsprechend können bei dem Markierungskörper gemäß Fig. 2 auch Verbindungselemente 22 zum Verbinden der Längsenden 14 und 16 vorgesehen sein oder eines der Längsenden 14 oder 16 kann aufgeweitet sein, so wie dies beispielsweise in den Figuren 1b, 3b oder 5b gezeigt ist.

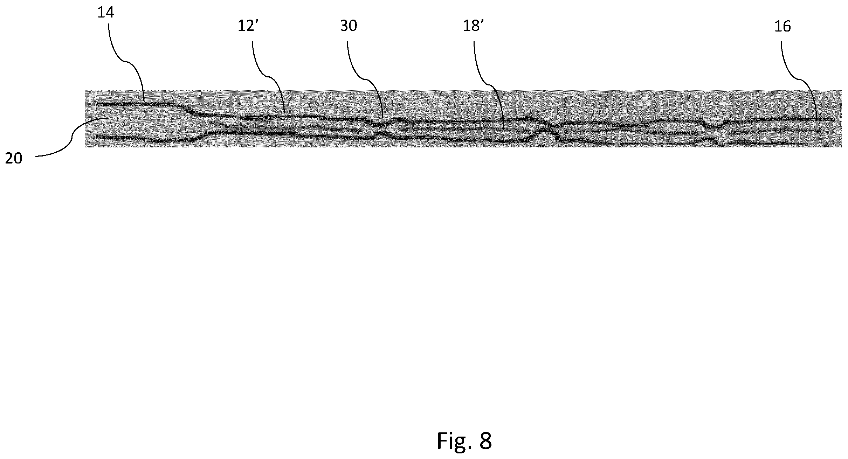

Um einen schlauchartigen Körper 12' auch ohne Werkzeug auf eine gewünschte Länge kürzen zu können, kann dieser Sollbruchstellen 30 aufweisen, so wie dies an dem in **Fig. 8** dargestellten Beispiel gezeigt ist. Der in Fig. 8 dargestellte schlauchartige Körper 12' kann durch Zusammenstecken seiner freien Längsenden 14 und 16 genauso zu einem Markierungskörper 10 geformt werden, wie die schlauchartigen Körper 12 aus den Figuren 1-5. Hierzu ist ein Längsende 14 entsprechend aufgeweitet, sodass das andere Längsende 16 in das aufgeweitete Längsende 14 eingesteckt werden kann.

In Bezug auf die Markierungselemente 18 zeigt Fig. 8, dass diese anstelle der Form relativ kurzer Metallringe auch als etwas längere Metallstäbe 18' ausgebildet sein können, die in entsprechende Lumina des schlauchartigen Körpers 12' eingesetzt sind bzw. in das Trägermaterial des schlauchartigen Körpers 12' eingegossen sind. Auch hier ist das Trägermaterial des schlauchartigen Körpers 12' vorzugsweise ein bioresorbierbarer Kunststoff.

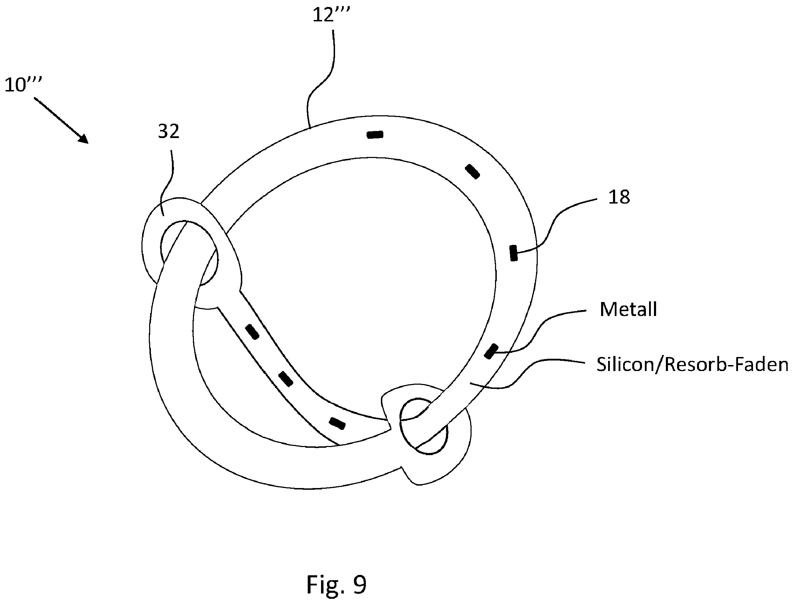

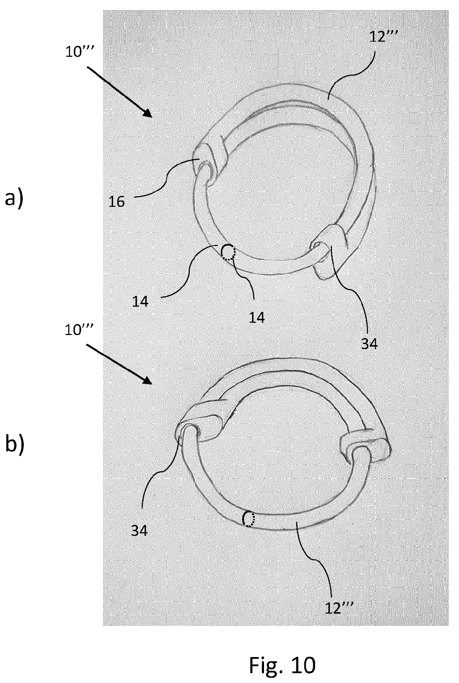

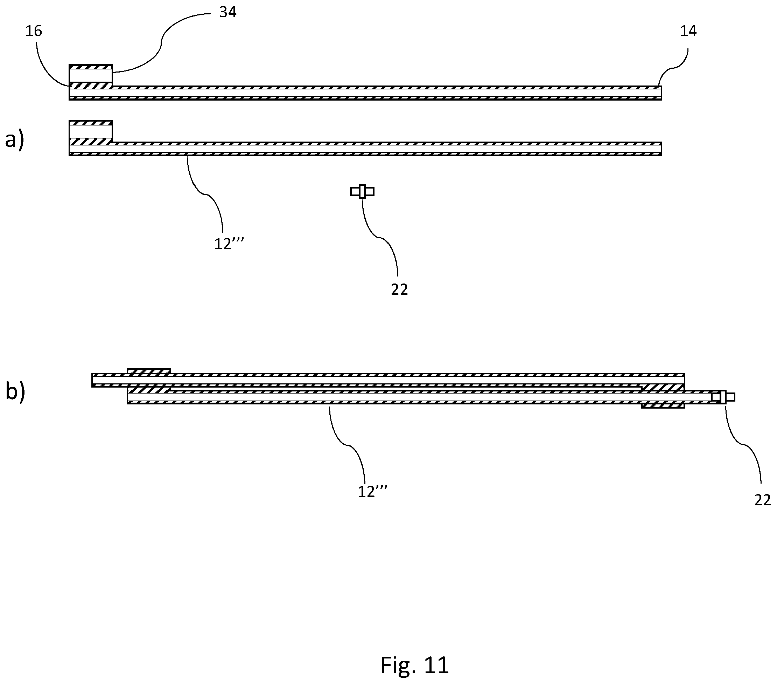

Um eine praktisch stufenlose Anpassung eines Markierungskörpers 10‴ an dem jeweiligen Implantationsort zu erlauben, kann auch ein schlauchartiger Körper 12‴ vorgesehen sein, der an einem Längsende oder an beiden Längsenden eine Schlaufe 32 **(****Figur 9****)** oder eine Hülse 34 **(****Figuren 10a, 10b****,** **11a** und **11b****)** aufweist, durch die der schlauchartige Körpers 12‴ hindurchgesteckt werden kann, um so eine verschiebliche Verbindung zwischen dem jeweiligen Längsende des schlauchartigen Körpers 12‴ und dem übrigen Schlauchkörper 12‴ herzustellen. Markierungskörper 10‴, wie sie beispielsweise in den Figuren 10a und b dargestellt sind, können aus zwei schlauchartigen Körpern 12‴ und einem Verbindungselement 22 zusammengesetzt sein. Dies ist in den Figuren 11a und 11b dargestellt. Fig. 11a zeigt zwei schlauchartige Körper 12‴ und ein Verbindungselement 22. Fig. 11b zeigt, wie zunächst die schlauchartigen Körper 12‴ so zusammengesteckt werden können, dass sie in den Hülsen 34 an ihrem jeweiligen Längsende 16 gleiten können. Die jeweils anderen Längsenden 14 der schlauchartigen Körper 12‴ können dann mithilfe des Verbindungelements 22 so miteinander verbunden werden, dass ein ringartiger, in der Weite verstellbarer Markierungskörper 10‴ entsteht, wie er in Figuren 10a oder 10b abgebildet ist. Der stufenlos einstellbare Durchmesser dieses Markierungskörpers 10‴ kann vorzugsweise durch einen nicht dargestellten Klemmmechanismus an mindestens einer Schlaufe 32 oder Hülse 34 oder in Stufen fixiert werden, bei denen ein Element an der Schlaufe mittels Formschluss an dem Bereich zwischen den Schlaufen fixiert wird.

Der Einfachheit halber nicht in allen Figuren dargestellt sind Markierungselemente 18, die bei den Ausführungsformen gemäß der Figuren 9 bis 11 genauso vorgesehen sind, wie bei den Ausführungsformen gemäß der Figuren 1 bis 5.

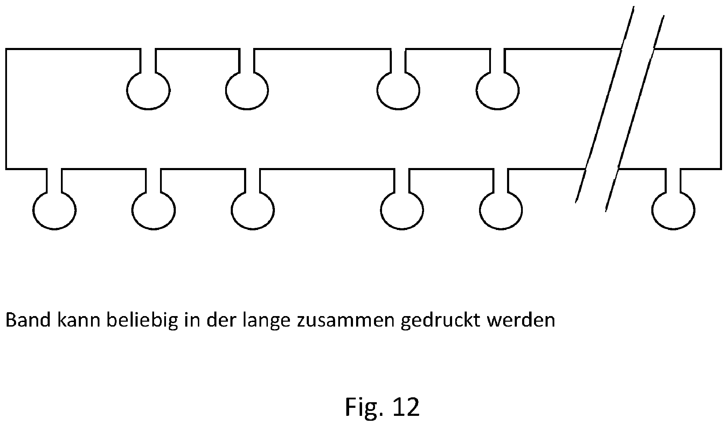

**Fig. 12** zeigt wiederum eine weitere Variante, wobei anstelle eines schlauchartigen Körpers ein Körper vorgesehen ist, der Vorsprünge 40 und entsprechend passende Ausnehmungen 42 aufweist, die nach Art von Teilen eines Puzzles miteinander verbunden werden können, um einen Markierungskörper herzustellen, der einen gewünschten Durchmesser aufweist. Der Einfachheit halber nicht in Figur 12 dargestellt sind Markierungselemente 18, die bei der Ausführungsform gemäß der Figur 12 genauso vorgesehen sind, wie bei den Ausführungsformen gemäß der Figuren 1 bis 5.

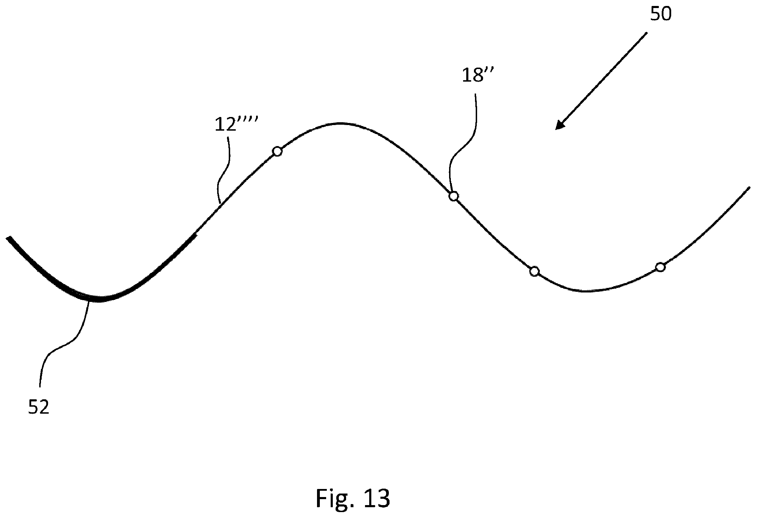

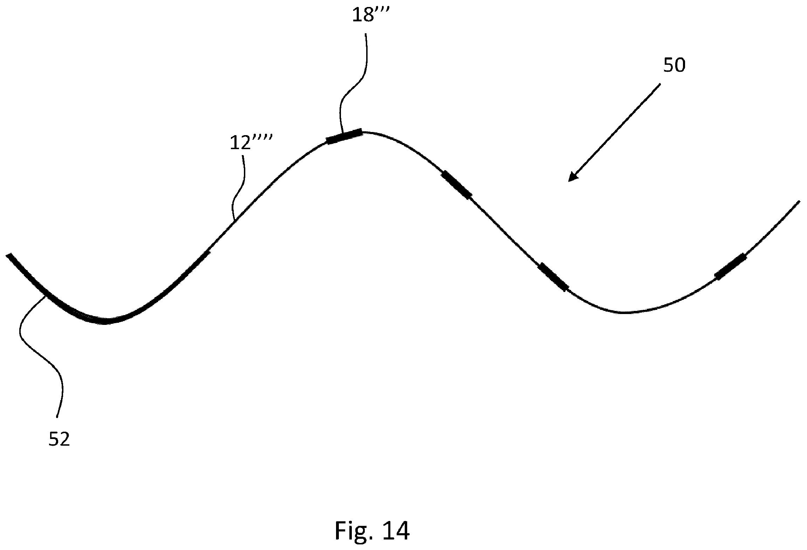

**Figuren 13** und **14** zeigen, dass anstelle solcher Markierungskörper 10, die aus schlauchartigen Körpern 12 zusammengesetzt sind, auch fadenartige Markierungskörper 50 vorgesehen sein können, die an einem Längsende nach Art einer gebogenen Metallnadel 52 gestaltet sind.

Außerdem ist den Figuren 13 und 14 zu entnehmen, dass die Markierungselemente 18 mit verschiedenen Formen vorgesehen sein können. Die Markierungselemente 18‴ in Figur 13 sind kugelförmig und außen auf dem fadenförmigen Körper 12′‴ fixiert. Die Markierungselemente 18′‴ in Figur 14 sind spindelförmig und ebenfalls außen auf dem fadenförmigen Körper 12′‴ fixiert.
- 10, 10', 10", 10‴: Markierungskörper
- 12, 12', 12‴: schlauchartiger Körper
- 14, 16: freie Längsenden des schlauchartigen Körpers
- 18: Markierungselemente
- 18': Metallstäbe als Markierungselemente
- 20: Lumen
- 22: Verbindungselement
- 24: Verbindungsenden
- 26: Markerelement
- 28: Vorsprung
- 30: Sollbruchstellen
- 32: Schlaufe
- 34: Hülse
- 40: Vorsprung
- 42: Ausnehmung
- 50: Markierungskörper
- 52: Metallnadel

Nachfolgend werden weitere, alternative Produktkonzepte erläutert.

### Produktkonzept: Mehrere Metallkugeln im Schlauch zum Auswerfen in das Tumorbett

### Beschreibung des Produktkonzeptes:

Das Konzept besteht aus mehreren Markersegmenten (z.B. 3, 5 oder bis zu 10), einer Nadel (in der die Segmente vorgeladen sind) und aus einem Auswerfer mit dem die Markersegmente aus der Nadel einzeln herausgeschoben werden.

Jedes Markersegment besteht aus einem Schlauch und einem Markerelement, der in dem Schlauch eingegossen ist.

Alle Markersegmente sind in einer Nadel vorgeladen - nacheinander - und können mit einem Auswerfer einzeln aus der Nadel herausgeworfen werden. Die Nadel hat einen Innendurchmesser I von z.B. 1-3 mm. Jedes Markersegment hat eine Länge a von z.B. 2-20 mm und dementsprechend ist die Nadel auch mehrere Zentimeter lang, so dass alle Markersegmente in der Nadel untergebracht werden können.

Der Schlauch besteht aus einem weichen bioresorbierenden Polymer (z.B. PLA, PLLA, Polyglycolsäure, Polycaprolacton, Poly-p-dioxanon, ε-Caprolacton, Evonik Resomer, oder ähnlich). Das Polymer soll so konfiguriert sein, dass es für ca. 6 Monate im Gewebe stabil ist und anschließend resorbiert oder es kann so konfiguriert sein, dass es für immer im Gewebe bleibt. Der Schlauch hat einen kleineren Durchmesser als der Innendurchmesser der Nadel. Der Schlauch kann aus einem Material sein, dass im Kontakt mit Wasser sein Volumen vergrößert (z.B. Hydrogel). Der Schlauch kann auch beschichtet sein, um z.B. Biokompatibilität zu gewährleisten. Oder der Schlauch kann beschichtet sein, um das Herausschieben aus der Kanüle zu gewährleisten.

Innerhalb des Schlauchs, z.B. in der Mitte jedes Segments, befindet sich ein Markerelement. Die Markerelemente zeichnen sich dadurch aus, dass sie auf Röntgen und CT-Bilder sichtbar sind. Hierzu sind biokompatible Metalle wie Gold, Titan oder Nitinol denkbar. Es sind auch Materialen wie Magnesium, Carbon, Calcium denkbar, die Röntgen-Strahlung absorbieren.

Die Markerelemente können beispielsweise die Form von kleinen Kugeln (Außendurchmesser b 0,5 - 3 mm) oder Zylindern (Außendurchmesser 0,5 - 3 mm, Länge 0,5 - 3 mm) aufnehmen. Auch andere geometrische Formen wie Würfel, Tetragon, Hexagon, Oktagon usw. sind denkbar.

Während der Anwendung wird ein Segment aus der Nadel an einer Stelle herausgeschoben und platziert. Die Nadel kann daraufhin zu einer anderen Stelle geführt werden und dort kann ein weiteres Segment platziert werden. Somit können mit einer Nadel die Segmente in dem gesamten Tumorbett verteilt werden.

### Zeichnung des Produktkonzeptes:

### Fig. 15 und Fig. 16

### Produktkonzept: Flexibles Netz

### Beschreibung des Produktkonzeptes:

Das flexible Netz lässt sich an die Form der Wundhöhle anpassen und wird an den Rändern der Wundhöhle per resorbierendes Nahtmaterial oder Gewebekleber (z.B. Fribrinkleber) befestigt. Somit ist sichergestellt, dass das Netz nicht migriert und die Ränder des Tumorbetts dauerhaft identifizierbar sind.

Das Netz sollte postoperativ nicht in der Brust tastbar sein. Das Netz besteht aus einem Polymer oder Copolymer (beispielweise aus Glycolid und Trimethylencarbonat, Polyglykolsäure-Caprolacton). Das Polymer soll so konfiguriert sein, dass es für ca. 6 Monate im Gewebe stabil ist und anschließend resorbiert. Auch wäre ein nicht resorbierendes Netz denkbar (z. B. aus Polypropylen, Polyester oder Polyamide). Sofern nicht resorbierbares Material verwendet wird, muss die Flexibilität hinsichtlich der angestrebten nicht Tastbarkeit konstruktiv erreicht werden. Das Netz ist in verschiedenen Größen (je nach Größe des Tumorbetts, Kantenlänge a = 5, 10, 15, 20 & 25 cm) erhältlich bzw. lässt sich je nach gewünschter Größe zurechtschneiden, sodass es für verschiedene Tumorbetten individuell anpassbar ist und unterschiedliche Größen annehmen kann. Der Abstand b der Maschen beträgt 0,5 bis 4,0 mm. Die flache Form ermöglicht Anpassungen an übliche onkoplastische Operationstechniken.

Eine Möglichkeit wäre, dass das Netz an sich röntgensichtbar (Beigabe von röntgensichtbarem Material, z.B. BaSO4, Tantal, Gold, Titan) und somit gut im CT sichtbar ist. Anhand von CT-Aufnahmen kann dann eine exakte Bestrahlung des Tumorbetts geplant und somit das umliegende gesunde Gewebe von der Bestrahlung verschont werden. Eine andere Möglichkeit wäre, dass das Netz mit röntgensichtbaren Markierungen (Marker aus Titan, Platin, Tantal, Gold in Abständen b von ca. 0,5 - 4,0 mm oder bis ein Vielfaches dieser Werte durch Auslassen von einigen Knotenpunkten) bestückt wird und die Mehrpunktmarkierungen als Referenz für die Bestrahlungsplanung genutzt werden. Dadurch, dass das Netz an die Form des Tumorbetts anpassbar ist, wird eine 3D Orientierung bei der Bestrahlungsplanung ermöglicht.

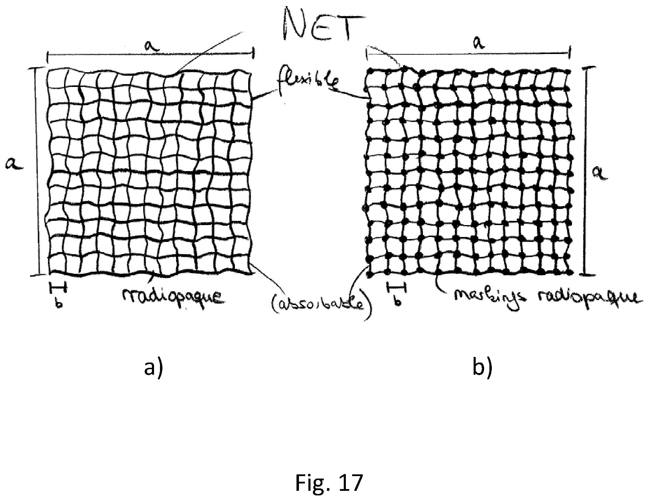

### Fig. 17: Zeichnung des Produktkonzeptes

### Produktkonzept: 3D-Matrix / Komprimierbarer Ball

### Beschreibung des Produktkonzeptes:

Die komprimierbare und postoperativ nicht tastbare 3D-Matrix / der komprimierbare und postoperativ nicht tastbare Ball (im folgenden Marker genannt) stellt ein (poröses) resorbierendes Gerüst dar und besteht aus einem Polymer (z.B. Polydioxanone, Kollagene oder Polyethylenglycol), das nach der Implantation die Regeneration des natürlichen Brustgewebes ermöglichen soll. Das Polymer soll so konfiguriert sein, dass es für ca. 6 Monate im Gewebe stabil ist und anschließend resorbiert. Zudem wird die Resorptionsrate auf das nachwachsende Gewebe abgestimmt. Sofern nicht resorbierbares Material (z.B. Silikon) verwendet wird, muss die Flexibilität hinsichtlich der angestrebten nicht Tastbarkeit konstruktiv erreicht werden. Die Herstellung des Markers könnte mit einem 3D-Druckverfahren erfolgen.

Der Marker ist in verschiedenen Größen (je nach Größe des Tumorbetts, Kantenlänge/ Durchmesser a = 1, 2, 3, 4 & 5 cm) erhältlich bzw. lässt sich je nach gewünschter Größe im OP zurechtschneiden/ -reißen (Sollbruchstellen). Um eine Migration des Markers zu verhindern, wird dieser am Rand des Tumorbetts befestigt.

Eine Möglichkeit wäre, dass der Marker an sich röntgensichtbar und somit gut im CT sichtbar ist (Beigabe von röntgensichtbarem Material, z.B. BaSO4, Tantal, Gold, Titan). Eine andere Möglichkeit wäre, dass der Marker mit röntgensichtbaren Markierungen bestückt wird, die in gewissen Abständen innerhalb und am Rand der Matrix/des Balls angeordnet sind (Marker aus Platin, Tantal, Gold in Abständen von ca. 0,5 1 cm). Die Mehrpunktmarkierungen bieten somit eine 3D Orientierung im Raum und dienen als Referenz für die Bestrahlungsplanung. Anhand von CT-Aufnahmen kann eine exakte Bestrahlung des Tumorbetts geplant und somit das umliegende gesunde Gewebe von der Bestrahlung verschont werden.

Zudem füllt der Marker die dreidimensionale Form der Tumorhöhle aus und verhindert somit, dass die Brust an dieser Stelle einfällt. Das Gewebe kann in den Marker einwachsen. Neben einer akkuraten Bestrahlungsplanung würde der Marker den kosmetischen Aspekt der Rekonstruktion unterstützen.

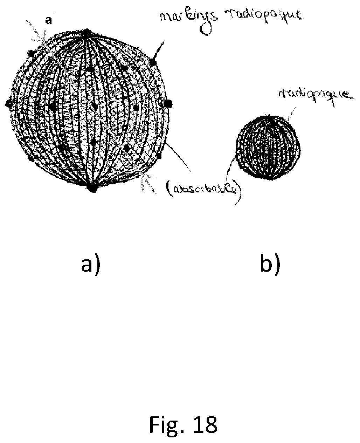

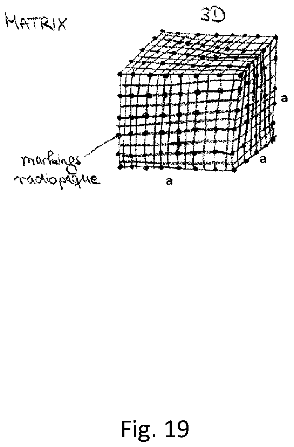

Fig. 18 und 19: Zeichnung des Produktkonzeptes:
Produktkonzept: Resorbierbare Magnesiumkugeln (als Röntgenmarker) verbunden mit einem resorbierbaren Faden/Draht

### Beschreibung des Produktkonzeptes:

Die resorbierbaren Magnesiumkugeln (als Röntgenmarker) (die Kugeln müssen nicht aus reinem Magnesium sein, sondern können auch aus einer Magnesiumlegierung bestehen, wie beispielweise Magnesium und Neodym als Zusatz, wodurch es fester wird und sehr gut formbar ist) sind mit einem resorbierbaren Faden/Draht (beispielsweise aus Magnesium/Magnesiumlegierung oder Polymilchsäure) verbunden. Der Faden/Draht ist plastisch verformbar und damit an die Wundhöhle anpassbar. Der Faden/Draht kann je nach gewünschter Größe/Länge zurechtgeschnitten werden (Gesamtlänge I des Fadens/Drahtes = 30 - 50 cm, Abstand a der Röntgenmarker = 1 - 3 cm). An dem vorderen Ende des Fadens/Drahtes ist eine Nadel befestigt, so dass der Faden/Draht inklusive Magnesiumkugeln direkt in die Wundhöhle eingenäht werden können. Dadurch ergibt sich ein dreidimensionales Konstrukt, wodurch die Wundhöhle zuverlässig lokalisiert und anhand dessen die Bestrahlungsplanung durchgeführt werden kann.

Die Resorptionszeit der Magnesiumkugeln kann mit unterschiedlichen Beschichtungen eingestellt werden. Die Beschichtung soll so konfiguriert sein, dass die Magnesiumkugeln für ca. 6 Monate im Gewebe stabil bleiben und anschließend resorbieren. Als Variante, falls Magnesium nicht röntgendicht genug ist, können auch Materialien wie Tantal oder Gold als Röntgenmarker genutzt werden.

### Zeichnung des Produktkonzeptes:

### Fig. 20

### Produktkonzept:

### Strahlentherapiefaden mit Metall-Segmenten

### Beschreibung des Produktkonzeptes:

Das Produktkonzept hat die Form eines Fadens in dem eine Vielzahl von Markerelementen eingebaut sind. Der Faden ist aus einem Material, der für chirurgische Fäden benutzt wird. Der Faden kann aus einem Material bestehen, der nach einer Zeit resorbiert (z.B. PLA, d.h. Polymilchsäure). Der Faden ist (plastisch) verformbar und damit an die Wundhöhle anpassbar. Der Faden kann eine Länge I von beispielweise 30-100 cm haben. Der Durchmesser des Fadens entspricht in etwa dem Durchmesser vom üblichen Nahtmaterial.

Die Markerelemente zeichnen sich dadurch aus, dass sie auf Röntgen und CT-Bilder sichtbar sind. Die Markerelemente zeichnen sich auch dadurch aus, dass sie in ihrer Flexibilität dem Faden ähneln und im Idealfall sogar den gleichen Durchmesser haben. Hierzu sind Faden, Litzen oder dünne Drähte aus biokompatiblen Metallen wie z.B. Gold, Titan oder Metalllegierungen wie z.B. Nitinol denkbar.

Jedes Markerelement kann beispielsweise eine Länge b von 5-20 mm haben. Es können beispielsweise 5-50 solcher Elemente entlang der gesamten Länge des Fadens verteilt sein. Die Markerelemente können gleichmäßig entlang des Fadens verteilt werden, oder sie sind eher ungleichmäßig verteilt.

Um den Strahlentherapiefaden herstellen zu können, werden zum Beispiel die Markerelemente mit Fadensegmenten durch Kleben, Verschweißen, Lasern usw. verbunden. Es können auch die Markerelemente in den Faden eingegossen oder reingedrückt werden.

An dem vorderen Ende ist eine Nadel befestigt, so dass der Strahlentherapiefaden inklusive Nitinol-Segmente direkt in die Wundhöhle eingenäht werden können. Dadurch ergibt sich ein dreidimensionales Konstrukt, wodurch die Wundhöhle zuverlässig lokalisiert und anhand dessen die Bestrahlungsplanung durchgeführt werden kann.

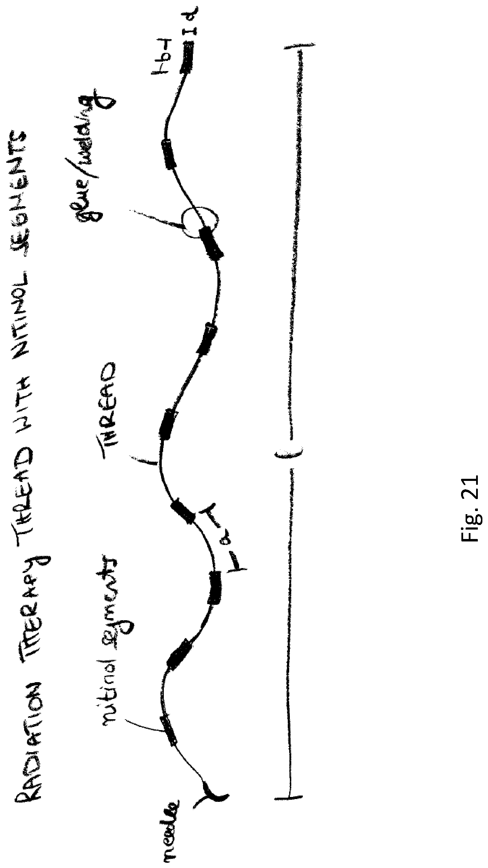

### Zeichnung des Produktkonzeptes: Fig. 21

### Produktkonzept: Silikonmarker/ Hydrogelmarker

### Beschreibung des Produktkonzeptes:

a.) Silikon als Grundmaterial:
   (als Implantatmaterial als Silikonschaum erhältlich, kann aber auch im spritzgussähnlichen Verfahren hergestellt werden)
   Formen: (je nach Tumorbettgröße im Durchmesser a = 1, 2, 3, 4 & 5 cm)
      - 2D- oder 3D-Stern oder
      - Spirale (ähnlich der Spirale vom Somatex-Lungenmarker / BioZorb) oder
      - Spiralband /-schnurr (wenn durchgehend ein gleiches Profil gewählt wird könnte ggf. durch einen kleinen chirurgischen Eingriff das Implantat entfernt werden)
   Röntgendicht durch:
      - kleine Metallteile ggf. Tantal, Gold, ... oder
      - als Zugabe von BaSO4 oder sonstigen Beimengungen in die Silikongrundmasse vor der Polymerisation
   Umsetzungsidee:
      Beispielweise ein 3D-Silikonstern: bestehend aus vier Zacken, die so ausgerichtet sind, dass zwischen jeder der vier Spitzen der gleiche Abstand vorhanden ist (dies könnte die Strahlentherapieplanung ggf. einfacher gestalten). An den Spitzen sind röntgendichte Markierungen befestigt, die als Referenz für die Bestrahlungsplanung dienen und eine 3D-Orientierung ermöglichen. Durch das Silikon wird sichergestellt, dass der Stern postoperativ nicht in der Brust tastbar ist. Zudem ist der Marker in verschiedenen Größen (je nach Größe des Tumorbetts) erhältlich. Um eine Migration des Markers zu verhindern, wird der Stern (an seinen Spitzen) am Rand des Tumorbetts befestigt.
b.) Hydrogel als Grundmaterial:
   (nur als langsam resorbierbare Variante mit geringen Quellfaktor ggf. auf PMMA Basis oder Zytosan)
   Formen: (je nach Tumorbettgröße im Durchmesser a = 1, 2, 3, 4 & 5 cm)
      - 2D oder 3D Stern oder
      - Spirale (ähnlich Spirale vom Somatex-Lungenmarker / BioZorb) oder
      - Spiralband /-schnurr
   Röntgendicht durch:
      - kleine Metallteile ggf. Tantal, Gold, ... oder
      - als Zugabe von BaSO4 oder sonstigen Beimengungen
   Umsetzungsidee:
      Siehe Beispiel Silikonstern, nur mit Hydrogel
   Zeichnung des Produktkonzeptes:

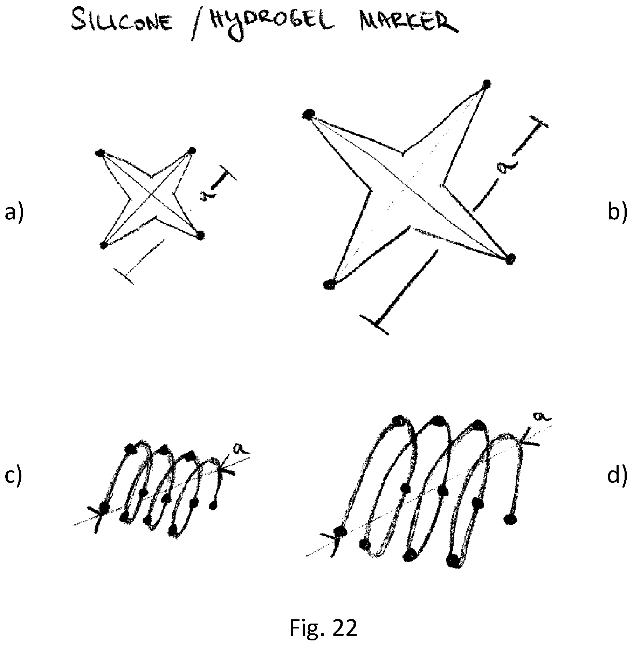

      Fig. 22a) bis 22)d

## Patentansprüche

1. Markierungskörper (10) insbesondere für die Markierung von Brustgewebe insbesondere eines Tumorbettes für eine Strahlentherapie, wobei der Markierungskörper (10) einen wenigstens abschnittsweise schlauchartigen Körper (12) aus einem weich-elastischem Material aufweist, der mehrere radioopake Markierungselemente (18) trägt, wobei der wenigstens abschnittsweise schlauchartige Körper (12) derart ausgebildet ist, dass er einer äußeren, verformenden Kraft kaum Widerstand entgegensetzt, in Abwesenheit von äußeren Kräften jedoch zu seiner ursprünglichen Form zurückkehrt und wobei der wenigstens abschnittsweise schlauchartige Körper (12) zwei freie Längsenden (14, 16) aufweist, die lösbar miteinander verbunden werden können oder miteinander verbunden sind, **dadurch gekennzeichnet, dass** der wenigstens abschnittsweise schlauchartige Körper (12) in seinem Ausgangszustand mit unverbundenen freien Enden im entspannten Zustand grade ist und durch Verbinden seiner beiden freien Enden miteinander zu einem elastischen Ring geformt wird, der wenigstens annähernd eine Kreisform hat.

2. Markierungskörper (10) gemäß Anspruch 1, **dadurch gekennzeichnet, dass** die elastischen Kräfte, die der Markierungskörper (10) einer äußeren Kraft entgegensetzt kleiner als 1N auf 1mm Komprimierungsweg bei einem Markierungskörper mit 3 cm Außendurchmesser sind.

3. Markierungskörper (10) gemäß Anspruch 1 oder 2, **dadurch gekennzeichnet, dass** die beiden freien Längsenden (14, 16) des wenigstens abschnittsweise schlauchartigen Körpers (12) dazu ausgebildet sind, mittels einer Steckverbindung miteinander verbunden zu werden.

4. Markierungskörper (10) gemäß wenigstens einem der Ansprüche 1 bis 3, **dadurch gekennzeichnet, dass** der wenigstens abschnittsweise schlauchartige Körper (12) wenigstens an einem seiner freien Längsenden (14, 16) ein Lumen (20) aufweist, in das ein anderes freies Längsende (16, 14) des wenigstens abschnittsweise schlauchartigen Körpers (12) oder ein Verbindungselement (22) eingesteckt werden kann, um zwei freie Längsenden (14, 16) des wenigstens abschnittsweise schlauchartigen Körpers (12) miteinander zu verbinden.

5. Markierungskörper (10) gemäß wenigstens einem der Ansprüche 1 bis 4, **dadurch gekennzeichnet, dass** die Verbindung zwischen den vor dem Verbinden freien Längsenden (14, 16) des wenigstens abschnittsweise schlauchartige Körpers (12) eine Klemmverbindung ist, bei der wenigstens eines der miteinander verbundenen freien Längsenden (14, 16) des wenigstens abschnittsweise schlauchartigen Körper (12) in Vergleich zu dessen unverbundenem Zustand radial zumindest geringfügig aufgeweitet ist, um durch elastische Rückstellkrafte die Klemmwirkung zu erzielen.

6. Markierungskörper (10) gemäß wenigstens einem der Ansprüche 1 bis 5, **dadurch gekennzeichnet, dass** der wenigstens abschnittsweise schlauchartige Körper (12) des Lumen (20) oder zwei oder mehr Lumina (20) aufweist, die sich zusammengenommen über mehr als die Hälfte der Gesamtlänge des wenigstens abschnittsweise schlauchartigen Körpers (12) erstrecken.

7. Markierungskörper (10) gemäß wenigstens einem der Ansprüche 1 bis 7, **dadurch gekennzeichnet, dass** der wenigstens abschnittsweise schlauchartige Körper (12) vorzugsweise aus einem weichen bioresorbierbaren Polymer wie PLA, PLLA, Poly glycolsäure, Polycaprolacton, Poly-p-dioxanon, £-Caprolacton, Evonik Resomer oder ähnlich bestehtwobei das Polymer vorzugsweise so konfiguriert ist, dass es für mindestens 6 Monate im Gewebe stabil ist und anschlielsend resorbiert.

8. Markierungskörper (10) gemäß wenigstens einem der Ansprüche 1 bis 7, **dadurch gekennzeichnet, dass** die radioopaken, vorzugsweise metallischen Markierungselemente (18) in einem gleichmäßigen Abstand voneinander an dem wenigstens ab schnittsweise schlauchartigen Körper (12) angeordnet sind, wobei der Abstand vorzugsweise zwischen 1cm und 3cm betragt.

9. Markierungskörper (10) gemäß wenigstens einem der Ansprüche 1 bis 8, **dadurch gekennzeichnet, dass** die radioopaken Markierungselemente (18) als Metallzylinder ausgebildet sind und eine Lange sowie einen Durchmesser von weniger als 5mm haben und entweder jeweils in ein Lumen (20) des wenigstens abschnittsweise schlauchartigen Körpers (12) eigesetzt oder auf diesen von außen aufgeschoben sind, und/oder **dadurch gekennzeichnet, dass** die Gesamtlänge des wenigstens abschnittsweise schlauchartigen Körpers (12) zwischen 7,5 cm und 30 cm betragt.

10. Markierungskörper (10) gemäß wenigstens einem der Ansprüche 1 bis 9, **dadurch gekennzeichnet, dass** die Verbindung der freien Längsenden (14, 16) des wenigstens abschnittsweise schlauchartigen Körpers (12) nach Art einer Steckbuchse erfolgen gestaltet ist, bei der ein freies Längsende in das andere, im Durchmesser etwas erweiterte Längsende des wenigstens abschnittsweise schlauchartigen Körpers (12) gesteckt wird.

11. Markierungskörper (10) gemäß wenigstens einem der Ansprüche 1 bis 9, **dadurch gekennzeichnet, dass** die beiden Längsenden (14, 16) des wenigstens abschnittsweise schlauchartigen Körpers (12) den gleichen Durchmesser haben und werden mittels eines Verbindungselements (22), vorzugsweise eines Verbindungsstifts, zusammengesteckt werden.

12. Markierungskörper (10) gemäß Anspruch 11, **dadurch gekennzeichnet, dass** das Verbindungselement (22) aus einem bioresorbierbaren Kunststoff besteht, der härter ist, als das Material, aus dem der wenigstens abschnittsweise schlauchartige Körper (12) gebildet ist.

13. Markierungskörper (10) gemäß Anspruch 11, **dadurch gekennzeichnet, dass** das Verbindungselement (22) ein Verbindungsstift aus Metall ist, der als radioopakes Markierungselement (18) dienen.

14. Markierungskörper (10) gemäß wenigstens einem der Ansprüche 1 bis 12, **dadurch gekennzeichnet, dass** der wenigstens abschnittsweise schlauchartige Körper (12) Ösen oder Schlaufen (32) aufweist, um den Markierungskörper (10) im Tumorbett einnähen und eine Migration des Markierungskörpers (10) verhindern zu können, und/oder **dadurch gekennzeichnet, dass** der Markierungskörper (10) aus mehreren wenigstens abschnittsweise schlauchartigen Körpern (12) zusammengesetzt ist, die an ihren Längsenden (14, 16) miteinander verbunden sind.

15. Markierungskörper (10) gemäß wenigstens einem der Ansprüche 1 bis 14, **dadurch gekennzeichnet, dass** der wenigstens abschnittsweise schlauchartige Körper (12) Sollbruchstellen (30) aufweist, an denen der wenigstens abschnittsweise schlauchartige Körper ohne Zuhilfenahme eines Werkzeugs von Hand gekürzt werden kann, und/oder **dadurch gekennzeichnet, dass** der wenigstens abschnittsweise schlauchartige Körper (12) an seinen beiden Längsenden (14, 16) Schlaufen (32) aufweist, durch die der im wesentlichen schlauchartige Körper (12) selbst hindurchgeführt ist, so dass sich ein in seiner Weite verstellbarer Ring ergibt.

## Claims

1. Marker body (10), in particular for marking breast tissue, in particular a tumor bed, for radiotherapy, the marker body (10) comprising an at least partially tubular body (12) made of a soft-elastic material, which body carries a plurality of radiopaque marker elements (18), the at least partially tubular body (12) being designed such that it offers little resistance to an external, deforming force, but returns to its original shape in the absence of external forces, and the at least partially tubular body (12) having two free longitudinal ends (14, 16) which can be or are detachably connected to one another, **characterized in that** the at least partially tubular body (12) is straight in its initial state with unconnected free ends in the relaxed state and is formed into an elastic ring having at least an approximately circular shape by its two free ends being connected to one another.

2. Marker body (10) according to claim 1, **characterized in that** the elastic forces with which the marker body (10) opposes an external force are less than 1 N per 1 mm compression path for a marker body having an outer diameter of 3 cm.

3. Marker body (10) according to claim 1 or 2, **characterized in that** the two free longitudinal ends (14, 16) of the at least partially tubular body (12) are designed to be connected to one another by means of a plug connection.

4. Marker body (10) according to at least one of claims 1 to 3, **characterized in that** the at least partially tubular body (12) has a lumen (20) at least at one of its free longitudinal ends (14, 16), into which lumen another free longitudinal end (16, 14) of the at least partially tubular body (12) or a connecting element (22) can be inserted in order to connect two free longitudinal ends (14, 16) of the at least partially tubular body (12) to one another.

5. Marker body (10) according to at least one of claims 1 to 4, **characterized in that** the connection between the longitudinal ends (14, 16) of the at least partially tubular body (12), which are free before the connection, is a clamp connection, in which at least one of the interconnected free longitudinal ends (14, 16) of the at least partially tubular body (12) is radially expanded at least slightly compared with its unconnected state in order to achieve the clamping effect by elastic restoring forces.

6. Marker body (10) according to at least one of claims 1 to 5, **characterized in that** the at least partially tubular body (12) has the lumen (20) or two or more lumens (20) which together extend over more than half the total length of the at least partially tubular body (12).

7. Marker body (10) according to at least one of claims 1 to 7, **characterized in that** the at least partially tubular body (12) preferably consists of a soft bioresorbable polymer such as PLA, PLLA, polyglycolic acid, polycaprolactone, poly-p-dioxanone, £-caprolactone, Evonik Resomer or similar, the polymer preferably being configured such that it is stable in the tissue for at least 6 months and is subsequently resorbed.

8. Marker body (10) according to at least one of claims 1 to 7, **characterized in that** the radiopaque, preferably metal marker elements (18) are arranged at a uniform distance from one another on the at least partially tubular body (12), the distance preferably being between 1 cm and 3 cm.

9. Marker body (10) according to at least one of claims 1 to 8, **characterized in that** the radiopaque marker elements (18) are designed as metal cylinders and have a length and a diameter of less than 5 mm and are each either inserted into a lumen (20) of the at least partially tubular body (12) or pushed onto it from the outside, and/or **characterized in that** the total length of the at least partially tubular body (12) is between 7.5 cm and 30 cm.

10. Marker body (10) according to at least one of claims 1 to 9, **characterized in that** the connection of the free longitudinal ends (14, 16) of the at least partially tubular body (12) is designed in the manner of a plug socket, in which one free longitudinal end is plugged into the other, slightly enlarged longitudinal end of the at least partially tubular body (12).

11. Marker body (10) according to at least one of claims 1 to 9, **characterized in that** the two longitudinal ends (14, 16) of the at least partially tubular body (12) have the same diameter and are plugged together by means of a connecting element (22), preferably a connecting pin.

12. Marker body (10) according to claim 11, **characterized in that** the connecting element (22) consists of a bioresorbable plastics material which is harder than the material from which the at least partially tubular body (12) is formed.

13. Marker body (10) according to claim 11, **characterized in that** the connecting element (22) is a connecting pin made of metal, which serves as a radiopaque marker element (18).

14. Marker body (10) according to at least one of claims 1 to 12, **characterized in that** the at least partially tubular body (12) has eyelets or loops (32) in order to make it possible to sew the marker body (10) into the tumor bed and to prevent migration of the marker body (10), and/or **characterized in that** the marker body (10) is composed of a plurality of at least partially tubular bodies (12) which are connected to one another at their longitudinal ends (14, 16).

15. Marker body (10) according to at least one of claims 1 to 14, **characterized in that** the at least partially tubular body (12) has predetermined breaking points (30) at which the at least partially tubular body can be shortened by hand without the aid of a tool, and/or **characterized in that** the at least partially tubular body (12) has loops (32) at its two longitudinal ends (14, 16) through which the substantially tubular body (12) itself is passed, so that a ring having an adjustable width is produced.

## Revendications

1. Corps de marquage (10), en particulier pour le marquage de tissu mammaire, en particulier d'un lit tumoral pour une radiothérapie, dans lequel le corps de marquage (10) présente un corps tubulaire au moins dans certaines sections (12) en un matériau souple et élastique qui porte plusieurs éléments de marquage radio-opaques (18), dans lequel le corps tubulaire au moins dans certaines sections (12) est conçu de telle sorte qu'il n'oppose pratiquement aucune résistance à une force extérieure de déformation, mais revient à sa forme initiale en l'absence de forces extérieures, et dans lequel le corps tubulaire au moins dans certaines sections (12) présente deux extrémités longitudinales libres (14, 16) qui peuvent être reliées l'une à l'autre ou sont reliées l'une à l'autre de manière amovible, **caractérisé en ce que** le corps tubulaire au moins dans certaines sections (12) est droit dans son état initial avec des extrémités libres non reliées à l'état détendu et est formé en reliant ses deux extrémités libres l'une à l'autre en formant un anneau élastique qui possède au moins approximativement une forme circulaire.

2. Corps de marquage (10) selon la revendication 1, **caractérisé en ce que** les forces élastiques que le corps de marquage (10) oppose à une force extérieure sont inférieures à 1 N sur 1 mm de trajet de compression pour un corps de marquage de 3 cm de diamètre extérieur.

3. Corps de marquage (10) selon la revendication 1 ou 2, **caractérisé en ce que** les deux extrémités longitudinales libres (14, 16) du corps tubulaire au moins dans certaines sections (12) sont conçues pour être reliées l'une à l'autre au moyen d'une liaison par enfichage.

4. Corps de marquage (10) selon au moins l'une des revendications 1 à 3, **caractérisé en ce que** le corps tubulaire au moins dans certaines sections (12) présente, au moins à l'une de ses extrémités longitudinales libres (14, 16), une lumière (20) dans laquelle peut être enfichée une autre extrémité longitudinale libre (16, 14) du corps tubulaire au moins dans certaines sections (12) ou un élément de liaison (22), afin de relier entre elles deux extrémités longitudinales libres (14, 16) du corps tubulaire au moins dans certaines sections (12).

5. Corps de marquage (10) selon au moins l'une des revendications 1 à 4, **caractérisé en ce que** la liaison entre les extrémités longitudinales libres (14, 16) avant la liaison du corps tubulaire au moins dans certaines sections (12) est une liaison par serrage avec laquelle au moins l'une des extrémités longitudinales libres (14, 16) reliées entre elles du corps tubulaire au moins dans certaines sections (12) est au moins légèrement élargie radialement par rapport à son état non relié, afin d'obtenir l'effet de serrage par des forces de rappel élastiques.

6. Corps de marquage (10) selon au moins l'une des revendications 1 à 5, **caractérisé en ce que** le corps tubulaire au moins dans certaines sections (12) présente la lumière (20) ou deux lumières (20) ou plus qui s'étendent conjointement sur plus de la moitié de la longueur totale du corps tubulaire au moins dans certaines sections (12).

7. Corps de marquage (10) selon au moins l'une des revendications 1 à 7, **caractérisé en ce que** le corps tubulaire au moins dans certaines sections (12) est de préférence constitué d'un polymère biorésorbable souple tel que PLA, PLLA, acide polyglycolique, polycaprolactone, poly-p-dioxanone, £-caprolactone, résomère de type Evonik ou similaire, dans lequel le polymère est de préférence conçu de sorte qu'il est stable dans le tissu pendant au moins six mois et pour se résorber par la suite.

8. Corps de marquage (10) selon au moins l'une des revendications 1 à 7, **caractérisé en ce que** les éléments de marquage radio-opaques (18), de préférence métalliques, sont disposés à une distance régulière les uns des autres sur le corps tubulaire au moins dans certaines sections (12), dans lequel la distance est de préférence comprise entre 1 cm et 3 cm.

9. Corps de marquage (10) selon au moins l'une des revendications 1 à 8, **caractérisé en ce que** les éléments de marquage radio-opaques (18) sont réalisés sous forme de cylindres métalliques et possèdent une longueur ainsi qu'un diamètre inférieurs à 5 mm et sont soit respectivement insérés dans une lumière (20) du corps tubulaire au moins dans certaines sections (12), soit enfilés sur celui-ci depuis l'extérieur, **et/ou caractérisé en ce que** la longueur totale du corps tubulaire au moins dans certaines sections (12) est comprise entre 7,5 cm et 30 cm.

10. Corps de marquage (10) selon au moins l'une des revendications 1 à 9, **caractérisé en ce que** la liaison des extrémités longitudinales libres (14, 16) du corps tubulaire au moins dans certaines sections (12) est réalisée à la manière d'une douille enfichable pour laquelle une extrémité longitudinale libre est enfichée dans l'autre extrémité longitudinale, légèrement élargie en diamètre, du corps tubulaire au moins dans certaines sections (12).

11. Corps de marquage (10) selon au moins l'une des revendications 1 à 9, **caractérisé en ce que** les deux extrémités longitudinales (14, 16) du corps tubulaire au moins dans certaines sections (12) possèdent le même diamètre et sont enfichées l'une dans l'autre au moyen d'un élément de liaison (22), de préférence d'une broche de liaison.

12. Corps de marquage (10) selon la revendication 11, **caractérisé en ce que** l'élément de liaison (22) est constitué d'une matière plastique biorésorbable qui est plus dure que le matériau à partir duquel est formé le corps tubulaire au moins dans certaines sections (12).

13. Corps de marquage (10) selon la revendication 11, **caractérisé en ce que** l'élément de liaison (22) est une broche de liaison en métal qui sert d'élément de marquage radio-opaque (18).

14. Corps de marquage (10) selon au moins l'une des revendications 1 à 12, **caractérisé en ce que** le corps tubulaire au moins dans certaines sections (12) présente des œillets ou des boucles (32) afin de pouvoir coudre le corps de marquage (10) dans le lit tumoral et d'empêcher une migration du corps de marquage (10), **et/ou caractérisé en ce que** le corps de marquage (10) est composé de plusieurs corps tubulaires au moins dans certaines sections (12) qui sont reliés entre eux au niveau de leurs extrémités longitudinales (14, 16).

15. Corps de marquage (10) selon au moins l'une des revendications 1 à 14, **caractérisé en ce que** le corps tubulaire au moins dans certaines sections (12) présente des points de rupture cibles (30) au niveau desquels le corps tubulaire au moins dans certaines sections peut être raccourci à la main sans l'aide d'un outil, **et/ou caractérisé en ce que** le corps tubulaire au moins dans certaines sections (12) présente à ses deux extrémités longitudinales (14, 16) des boucles (32) à travers lesquelles le corps sensiblement tubulaire (12) lui-même est passé, de sorte qu'il en résulte un anneau dont la largeur est réglable.
